# EUROPEAN PATENT APPLICATION

(11) **EP 2 210 936 A1**
(43) Date of publication of application: **28.07.2010**
(21) Application number: 09001095.0
(22) Date of filing: 27.01.2009
(51) Int. Cl.: C12N 1/06

(54) **Processing and analysis of viscous liquid biological samples**

(71) Applicant: Curetis AG, 71088 Holzgerlingen (DE)
(72) Inventor: Klein, Matthias, 71034 Böblingen (DE)
(74) Representative: Schnappauf, Georg

(57) **Abstract**

The present invention provides a lysis buffer comprising a chaotropic agent and a reducing agent suitable for liquefaction of a highly viscous liquid biological sample, such as sputum, a use of said lysis buffer for the processing of a highly viscous liquid biological sample, methods for processing or analyzing a highly viscous liquid biological sample, or a method for detecting a pathogen within a highly viscous liquid biological sample. Furthermore, the present invention relates to a lysate comprising a highly viscous liquid biological sample and the lysis buffer according to the present invention, a ready-to-use reaction tube and a kit comprising the lysis buffer according to the present invention.

## Description

### TECHNICAL FIELD OF THE INVENTION

The invention relates to the processing and analysis of highly viscous liquid biological samples such as biological samples comprising sputum.

### BACKGROUND OF THE INVENTION

Diagnostic procedures often require the analysis of biological samples such as body fluids. In particular, nucleic acid based diagnostic methods are becoming more and more important. However, such methods generally require initial processing of the biological sample which may be time-consuming, laborious, and associated with the risk of contamination. For example, the diagnosis of tuberculosis involves the analysis of highly viscous liquid biological samples such as sputum, pus, pleural fluid, gastric aspirate, endotracheal aspirate, transtracheal aspirate, bronchoalveolar lavage, laryngeal swab, and nasopharyngeal swabs, which are usually inhomogeneous mixtures of many different components of different chemical and physical behavior.

Sputum consists of variable amounts of glycoproteins (mucins), saliva, immune cells, host tissue particles, released DNA, lipids, and proteins from lysed host tissue. Biochemical analyses have revealed that mucins MUC5AC and MUC5B secreted by cells lining the respiratory tract are the major gel-forming polymer components of airway mucus. Cysteine domains present on these mucins contribute to polymer formation and possibly interaction with neighboring mucin chains by disulfide bonding. Certain sputa can contain variable amounts of blood or residual food particles as contaminants. This results in a very extensive sample-to-sample variability of sputum composition ranging from homogeneous to multiphasic on the one side and liquid to highly viscous on the other side. Dependent of the disease state of individual patients, sputa can furthermore contain inflammatory pathogens, and certain sample components can be extremely pronounced, e.g., blood contamination due to lung inflammation or elevated viscosity due to an extensive DNA release for cystic fibrosis or bronchitis patients.

Because of the extensive sample heterogeneity processing of sputum samples such as DNA isolation from sputum for diagnostic purposes is rather challenging. For instance, accessibility and lysis of inflammatory pathogens can be less efficient if they are trapped in a solid and viscous environment. Furthermore, the wide variety of sputum components can lead to copurification of inhibitors for subsequent sensitive reactions like DNA amplification. Unlysed and unliquefied sputum components can interfere with subsequent DNA purification techniques commonly used, e.g., by membrane clogging during a silica membrane purification approach.

On the other hand, analysis of sputum samples is a standard diagnostic procedure for patients with suspected tuberculosis. The classical methods for diagnosis include examination of sputum smear under a microscope for acid-fast mycobacteria and microbiological analysis of cultured mycobacteria isolated from sputum which is the current gold standard for identification of pathogens and resistances in tuberculosis diagnosis. In addition, some molecular tests have been developed. Generally, all these diagnostic methods aiming at detection of mycobacteria in sputum samples require laborious sample processing for decontamination and liquefaction using enzymes such as proteases, lipases, DNases, or glycosidases, detergents, chaotropic agents, chelating agents, and reducing agents among others. However, some of these agents like SDS are known inhibitors of nucleic acid amplification and analysis methods. Furthermore, due to the high infection risk any treatment of tuberculosis suspected sputa requires an S3 environment with certified laminar flows and extensive protection measures to exclude any exposure of personnel to live bacteria. Thus, for molecular tests it would be of advantage to use sputum directly for nucleic acid diagnostics and circumvent the handling intensive decontamination and liquefaction procedures.

The present invention solves the above problems by providing a lysis buffer, uses thereof, and methods applying said lysis buffer which allow for a one step lysis of highly viscous liquid biological samples such as sputum for molecular diagnostic purposes. The inventors of the present invention surprisingly found that only a subset of the reagents expected to be required for processing of a highly viscous liquid biological sample such as sputum is required for sufficient liquefaction such that, for example, raw sputum that is treated with the lysis buffer according to the invention can be directly applied to nucleic acid isolation and/or molecular diagnostic procedures.

### SUMMARY OF THE INVENTION

The present invention provides a buffer for lysing a highly viscous liquid biological sample comprising at least one chaotropic agent and at least one reducing agent. Preferably, the lysis buffer does not contain proteases, DNases, glycosidases, and lipases. Preferably, the lysis buffer further comprises beads, preferably made of glass, ceramics, metal, or other solid and inert materials. In a preferred embodiment of the lysis buffer according to the present invention, the nature and concentration of the at least one chaotropic agent and/or the nature and concentration of the at least one reducing agent is such that liquefaction of a highly viscous liquid biological sample is achieved when the lysis buffer is mixed with the sample. Preferably, the chaotropic agent is selected from the group consisting of guanidinium thiocyanate, guanidinium isothiocyanate, guanidinium hydrochloride, guanidinium chloride, alkali thiocyanate, alkali isothiocyanate, alkali iodide, and alkali perchlorate. Preferably, the reducing agent is selected from the group consisting of dithiothreitol (DTT), N-acetyl-cysteine (NALC), beta-Mercaptoethanol, Tris(2-Carboxyethyl) phosphine (TCEP), and thioredoxin.

Furthermore, the present invention provides the use of the lysis buffer according to the present invention for processing a highly viscous liquid biological sample. Preferably, processing comprises liquefaction of the highly viscous liquid biological sample.

In another aspect, the present invention provides a method for processing a highly viscous liquid biological sample, comprising the steps of (a) mixing the sample with the lysis buffer according to the present invention, (b) optionally heating the mixture, and (c) optionally bead milling the mixture.

Furthermore, the present invention provides a method for analyzing a highly viscous liquid biological sample comprising the steps of (a) mixing the sample with the lysis buffer according the present invention, (b) optionally heating the mixture, (c) optionally bead milling the mixture, (d) applying the mixture to a nucleic acid amplification/analysis method.

In addition, the present invention relates to a method for detecting the presence of a pathogen in a highly viscous liquid biological sample comprising the steps of (a) mixing the sample with the lysis buffer according to the present invention, (b) optionally heating the mixture, (c) optionally bead milling the mixture, (d) applying the mixture to a nucleic acid amplification/analysis method that is suitable for detection of said pathogen.

In a preferred embodiment of the methods according to the present invention, the sample is untreated (e.g., not mixed with a reducing agent like sputolysin to reduce viscosity) before mixing it with the lysis buffer in step (a). Preferably, the methods according to the present invention further comprise the step of isolating a nucleic acid from the mixture of step (c). Preferably, said isolation is performed without the need for any further processing such as extraction using organic solvents or precipitation.

In another aspect, the present invention relates to a lysate of a highly viscous liquid biological sample comprising a highly viscous liquid biological sample and the lysis buffer according to the present invention.

Furthermore, the present invention provides a ready-to-use reaction tube containing the lysis buffer according to the present invention.

In addition, the present invention relates to a kit comprising the lysis buffer according to the present invention and an instruction leaflet containing instructions for using said lysis buffer for liquefaction of a highly viscous liquid biological sample.

In a particular preferred embodiment of the lysis buffer, the use, the methods, the lysate, and the kit according to the present invention, the highly viscous liquid biological sample is selected from the group consisting of a biological sample comprising sputum, pus, pleural fluid, gastric aspirate, endotracheal aspirate, transtracheal aspirate, bronchoalveolar lavage, laryngeal swab, and nasopharyngeal swabs.

### BRIEF DESCRIPTION OF THE DRAWINGS

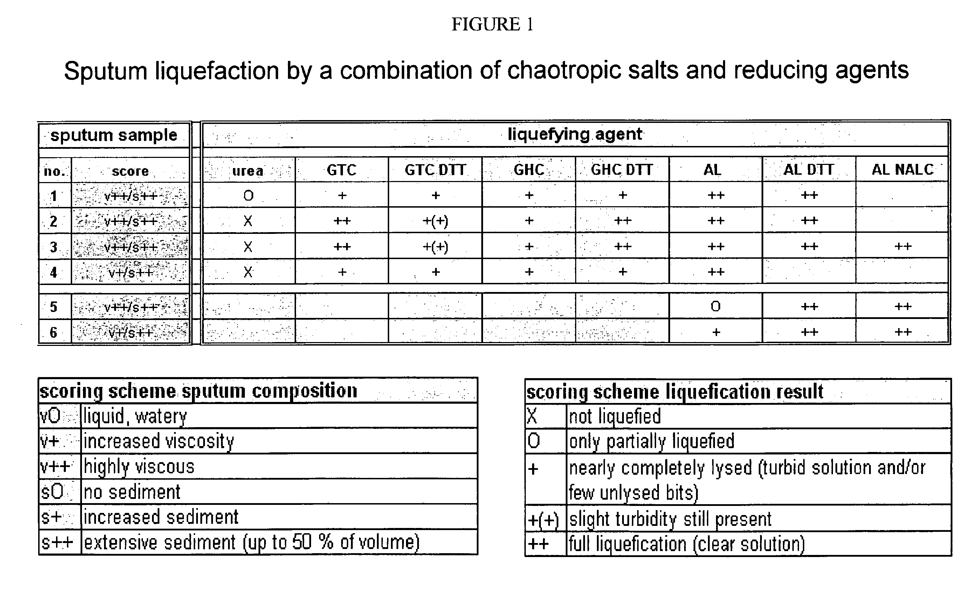

Figure 1: Sputum liquefaction by a combination of chaotropic salts and reducing agents. Untreated sputum samples were liquefied using variable lysis buffers containing different chaotropic agents and reducing agents without glass beads. The samples were heated to 96°C for 15 minutes and viscosity was scored before and after treatment.

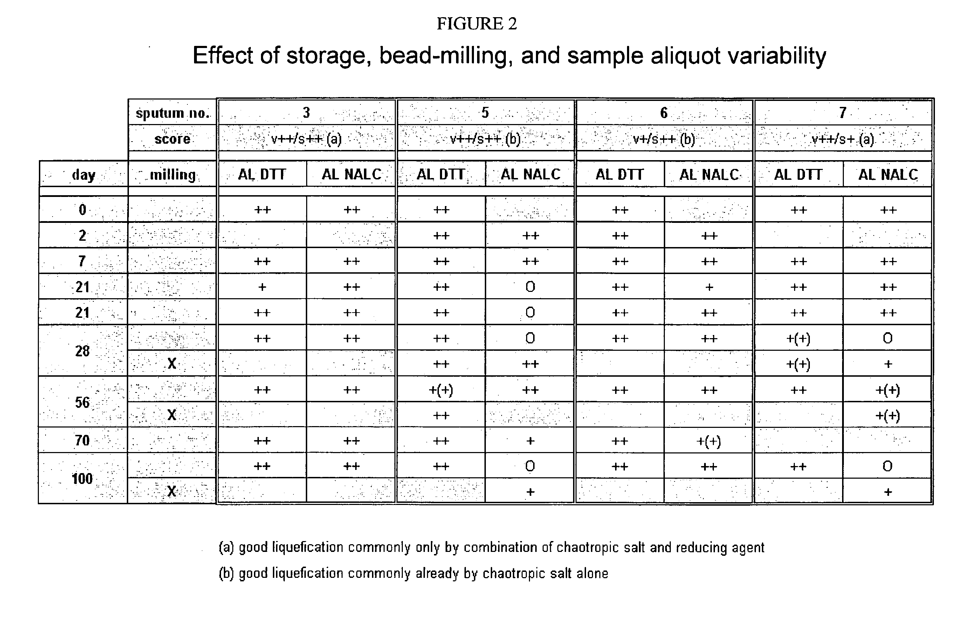

Figure 2: Effect of storage, bead milling, and sample aliquot variability. Untreated sputum samples were liquefied using lysis buffer 1 with DTT or NALC, with beads. Samples were heated at 96°C for 15 minutes. Some samples were bead milled for 5 minutes where indicated (X in column "milling"). Buffers have been stored at 15°C to 25°C for 0, 2, 7, 21, 28, 56, 70, or 100 days before the experiment. Viscosity was determined before and after treatment according to the scoring scheme shown in Figure 1.

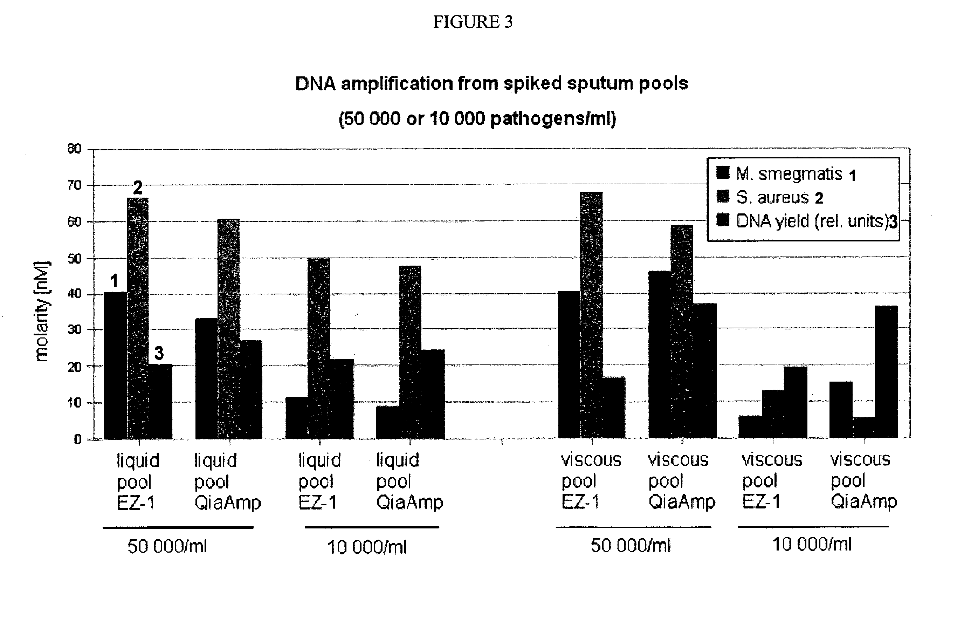

Figure 3: Performance and sensitivity test for pretreated sputum pools. Sputolysin pretreated sputum pools (5 samples from different patients for each pool) were spiked with *M. smegmatis* (50000 pathogens/ml or 10000 pathogens/ml) and liquefied using lysis buffer 1 with DTT and glass beads followed by DNA isolation as described in Examples 1 and 4. The isolated DNA was subjected to a PCR reaction using *M. smegmatis* specific primers. The diagram shows the molarity of amplified *M. smegmatis* DNA for sputum samples spiked with 50000 pathogens/ml and 10000 pathogens/ml as well as the relative yield of each preparation. Different DNA isolation procedures, i.e., silica membrane based (QIAamp) and magnetic beads (EZ-1) are compared.

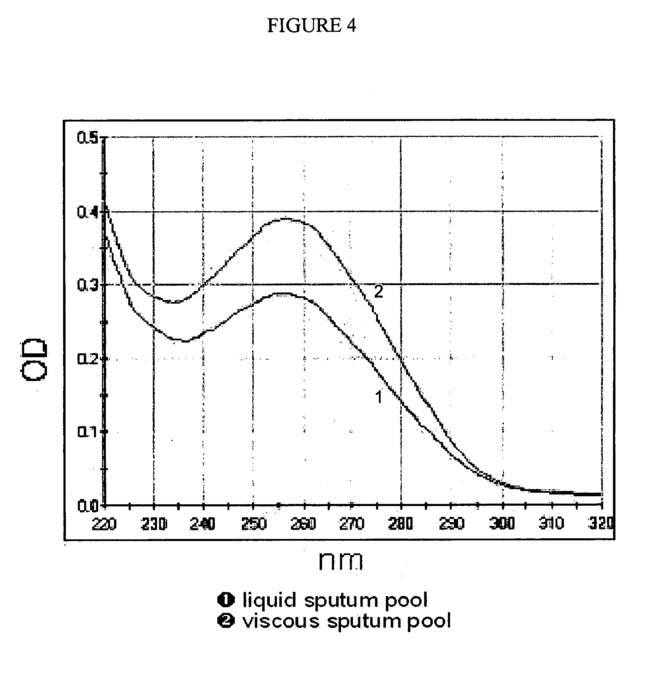

Figure 4: Spectrophotometric analysis of DNA isolated from pretreated sputum pools. The spectrum of DNA isolated from a liquid sputum pool is compared to the spectrum of DNA isolated from a viscous sputum pool.

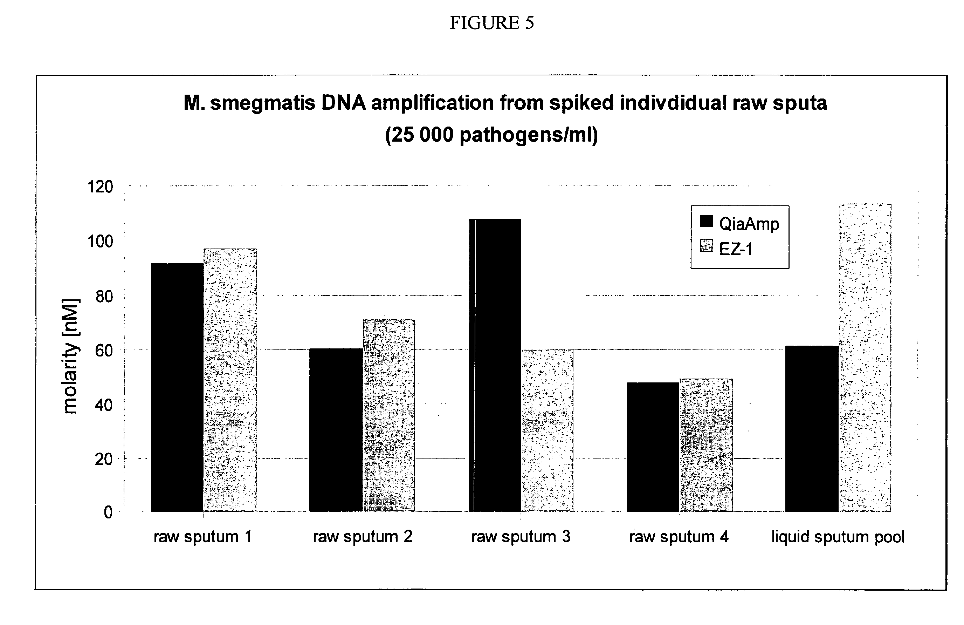

Figure 5: Performance and sensitivity test for untreated individual sputum samples. Individual untreated raw sputum samples were spiked with *M. smegmatis* (25000 pathogens/ml) and liquefied using lysis buffer 1 with DTT and glass beads as described in Examples 1 and 4. The isolated DNA was subjected to a PCR reaction. The diagram shows the molarity of amplified *M. smegmatis* DNA for sputum samples spiked with 25000 pathogens/ml. Different DNA isolation procedures, i.e., silica membrane based (QIAamp) and magnetic bead based (EZ-1) are compared. Untreated raw sputum samples 1 to 4 are compared with a pretreated liquid sputum pool.

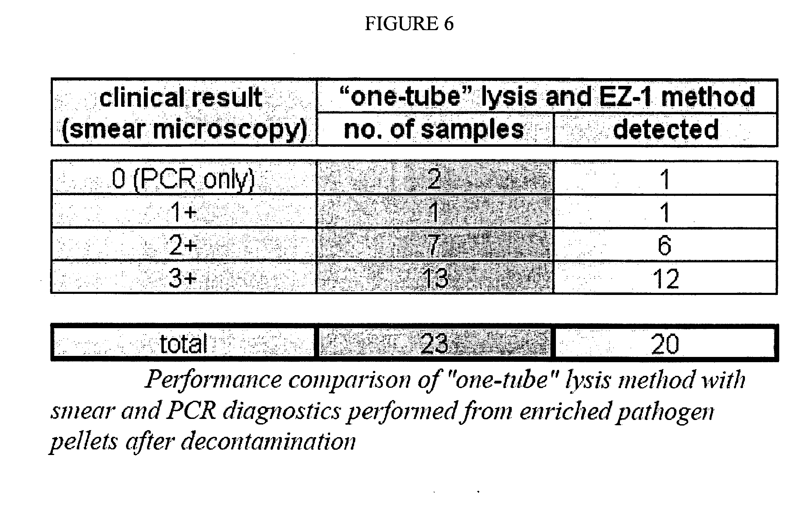

Figure 6: Performance comparison of the "one-tube" lysis method according to the present invention with classical diagnostic approaches for tuberculosis (smear microscopy and PCR performed from enriched pathogen pellets after decontamination). Four different sample categories were tested: 0: detected positive only by PCR (not by smear microscopy); 1+: low pathogen load (detected by smear microscopy); 2+: moderate pathogen load (detected by smear microscopy), 3+: high pathogen load (detected by smear microscopy). For this experiment, primers specific for the *Mycobacterium tuberculosis* complex were used.

### DETAILED DESCRIPTION OF THE INVENTION

Although the present invention is described in detail below, it is to be understood that this invention is not limited to the particular methodologies, protocols and reagents described herein as these may vary. It is also to be understood that the terminology used herein is for the purpose of describing particular embodiments only, and is not intended to limit the scope of the present invention which will be limited only by the appended claims. Unless defined otherwise, all technical and scientific terms used herein have the same meanings as commonly understood by one of ordinary skill in the art.

In the following, the elements of the present invention will be described. These elements are listed with specific embodiments, however, it should be understood that they may be combined in any manner and in any number to create additional embodiments. The variously described examples and preferred embodiments should not be construed to limit the present invention to only the explicitly described embodiments. This description should be understood to support and encompass embodiments which combine the explicitly described embodiments with any number of the disclosed and/or preferred elements. Furthermore, any permutations and combinations of all described elements in this application should be considered disclosed by the description of the present application unless the context indicates otherwise. For example, if in a preferred embodiment the chaotropic agent of the lysis buffer of the invention is guanidinium hydrochloride and in another preferred embodiment the reducing agent of the lysis buffer is dithiothreitol, it is a preferred embodiment of the present invention that guanidinium hydrochloride and dithiothreitol are present in the lysis buffer according to the present invention.

Preferably, the terms used herein are defined as described in "A multilingual glossary of biotechnological terms: (IUPAC Recommendations)", H.G.W. Leuenberger, B. Nagel, and H. Kölbl, Eds., Helvetica Chimica Acta, CH-4010 Basel, Switzerland, (1995).

The practice of the present invention will employ, unless otherwise indicated, conventional methods of chemistry, biochemistry, and recombinant DNA techniques which are explained in the literature in the field (cf., e.g., Molecular Cloning: A Laboratory Manual, 2nd Edition, J. Sambrook et al. eds., Cold Spring Harbor Laboratory Press, Cold Spring Harbor 1989).

Throughout this specification and the claims which follow, unless the context requires otherwise, the word "comprise", and variations such as "comprises" and "comprising", will be understood to imply the inclusion of a stated member, integer or step or group of members, integers or steps but not the exclusion of any other member, integer or step or group of members, integers or steps. As used in this specification and the appended claims, the singular forms "a", "an", and "the" include plural referents, unless the content clearly dictates otherwise.

Several documents are cited throughout the text of this specification. Each of the documents cited herein (including all patents, patent applications, scientific publications, manufacturer's specifications, instructions, etc.), whether *supra* or *infra*, are hereby incorporated by reference in their entirety. Nothing herein is to be construed as an admission that the invention is not entitled to antedate such disclosure by virtue of prior invention.

A "lysis buffer" in the context of the present invention is suitable for lysis of cells with the purpose of analyzing the contents of the cells. Preferably, the lysis buffer according to the present invention is suitable for the lysis of bacterial cells, more preferably for the lysis of mycobacteria. The lytic properties of the lysis buffer according to the present invention may require heating of the sample to be lysed in said lysis buffer depending on the type of cells to be lysed.

The term "chaotropic agent" refers to an agent which disrupts the three dimensional structure of macromolecules such as proteins, DNA, or RNA and denatures them. Chaotropic agents interfere with stabilizing intra-molecular interactions mediated by non-covalent forces such as hydrogen bonds, van der Waals forces, and hydrophobic effects. Chaotropic ions are, for example, guanidinium, barium, thiocyanate, iodide, and perchlorate, according to the so called Hofmeister series (cations: NH₄⁺ > Rb⁺ > K⁺ > Na⁺ > Cs⁺ > Li⁺ > Mg²⁺ > Ca²⁺ > Ba²⁺ > guanidinium; anions: PO₄³⁻ > SO₄²⁻ > HPO₄²⁻ > acetate > citrate > tartrate > Cl⁻ > Br⁻ > NO₃⁻ > ClO₃⁻ > ClO₄⁻ > I⁻ > SCN⁻). In the Hofmeister series, cations and anions on the left are said to be "kosmotropic" (or antichaotrope) and increase the strength of hydrophobic interactions. Ions on the right are "chaotropic" and tend to weaken hydrophobic interactions. For all aspects of the invention, the chaotropic agent contains at least one cation of the Hofmeister series which is further to the right in the series than calcium or at least one anion of the Hofmeister series which is further to the right in the series than the chlorate (ClO₃⁻) anion. For example, the chaotropic agent in the context of the present invention may contain one of the following: barium, guanidinium, perchlorate, iodide, thiocyanate, or isothiocyanate. For example, the chaotropic agent in the context of all aspects of the present invention may be guanidinium thiocyanate, guanidinium isothiocyanate, guanidinium hydrochloride, guanidinium chloride, alkali thiocyanate, alkali isothiocyanate, alkali iodide, or alkali perchlorate. In this context, the alkali ion is preferably potassium or sodium.

A "reducing agent" in the context of the present invention is any agent that is capable of breaking up a disulfide bond in or within macromolecules such as proteins. For all aspects of the present invention the reducing agent may be dithiothreitol (DTT), N-acetyl-cysteine (NALC), beta-Mercaptoethanol, Tris(2-Carboxyethyl) phosphine (TCEP), or thioredoxin. Preferably, the reducing agent is DTT.

The terms "wherein the nature and concentration of the chaotropic agent is such that liquefaction of a highly viscous liquid biological sample is achieved" and "wherein the nature and concentration of the reducing agent is such that liquefaction of a highly viscous liquid biological sample is achieved" mean that the chaotropic agent and/or the reducing agent and their concentrations are chosen based on their ability to liquefy a highly viscous liquid biological sample. In particular, this means that the highly viscous liquid biological sample exhibits a higher viscosity before treatment with the chaotropic agent and/or the reducing agent compared to the viscosity after said treatment. Preferably, the viscosity before treatment is at least 2-, 3-, 4-, 5-, 6-, 7-, 8-, 9-, or 10-fold higher than after treatment, most preferably the viscosity before treatment is 10-fold higher than after the treatment. Thus, in order to determine the nature of the chaotropic agent and/or the reducing agent as well as the appropriate concentrations thereof, the skilled person may incubate a highly viscous liquid biological sample, such as sputum, with the candidate chaotropic agent and/or reducing agent at various concentrations, for example, at 1 M, 2 M, 3 M, 4 M, 5M, and 6 M for the chaotropic agent and/or at 1 mM, 5 mM, 10 mM, 25 mM, 50 mM, and 100 mM for the reducing agent, for a certain period of time, such as for 10 minutes, 20 minutes, or 30 minutes, and determine the viscosity of the sample before and after the treatment. Then the chaotropic agent and/or the reducing agent are chosen in concentrations suitable to liquefy the highly viscous liquid biological sample as specified above. For example, guanidinium hydrochloride and guanidinium thiocyanate in a concentration of 2 M to 6 M are able to liquefy a highly viscous liquid biological sample such as sputum.

The term "processing" in the context of the present invention refers in general to every treatment that comprises a change in one or more physical properties of a sample being processed when said physical property/properties is/are determined before and after the treatment/processing. Preferably, "processing" in the sense of the present invention comprises liquefaction of a sample such that the viscosity of the sample is reduced by the processing procedure. It is particularly preferred that "processing" comprises lysis of the sample, meaning the disintegration of cells present in the sample. Such cells may be prokaryotic or eukaryotic cells, for example, bacterial cells, yeast cells, fungal cells, animal cells, mammalian cells etc, wherein processing may lead to lysis of all cells in the sample or only of a specific type of cells or a subset of bacteria. In the context of the present invention, it is most preferred that processing comprises the lysis of mycobacteria.

The term "liquefaction" in the context of the present invention means that the viscosity of a highly viscous liquid biological sample before liquefaction is higher, preferably at least 2-fold higher compared to the viscosity after liquefaction. Preferably, the viscosity of the sample is at least 3-fold higher before liquefaction compared to the viscosity after liquefaction. More preferably, the viscosity of the sample is at least 5-fold, most preferably at least 10-fold higher before liquefaction compared to the viscosity after liquefaction.

"Viscosity" in the context of the present invention means dynamic viscosity, i.e., η, which is measured in kg·m⁻¹·s⁻¹ = Pa·s. Another common unit for dynamic viscosity is centipoise (cP), wherein 1 cP equals 1 mPa·s. Water at 20°C has a viscosity of 1.0020 cP. Some examples for materials with higher viscosities are: blood at 37°C = 4-25 mPa·s, olive oil = ∼ 100 mPa·s, honey = 2000-10000 mPa·s, chocolate syrup = 10000-25000 mPa·s, molten chocolate = 45000-130000 mPa·s, and peanut butter = ∼ 250000 mPa·s. The skilled person is well aware of how to determine the viscosity of a sample. For example, instruments for the measurement of viscosity, i.e., viscometers, are commercially available.

A "highly viscous liquid biological sample" in the context of the present invention means a material with a high viscosity, preferably a viscosity of at least 1 x 10⁴ mPa·s, wherein the sample is still considered "liquid" if part of the material, preferably more than 50% of the material is liquid. Part of the sample material, preferably less than 50%, preferably less than 40%, more preferably less than 30%, most preferably less than 20% of the material may be solid material such as tissue chunks. Thus, in the context of the present invention, semi-solid materials, such as peanut butter, are also considered liquid. In the context of the present invention, the highly viscous liquid biological sample is preferably derived from an animal, preferably from a human, and preferably the sample is for diagnosing a disease, for example, by detecting the presence of a pathogen. Most preferably, the "highly viscous liquid biological sample" in the context of the present invention is a sputum sample.

The term "untreated" in the context of the present invention means that the highly viscous liquid biological sample is not treated by application of one or more chemical agent(s) or physical forces such as temperature or shearing forces, or any other procedures such as centrifugation, filtering, or sieving. For all aspects of the present invention it is preferred that the highly viscous liquid biological sample is untreated before it is mixed with the lysis buffer according to the present invention. However, in the event the highly viscous liquid biological sample is stored for a certain period of time, for example, for more than 8 hours, before mixing it with the lysis buffer according to the invention, it is preferred that the highly viscous liquid biological sample is stored at a temperature below 0°C, preferably below -5°C, more preferably below -10°C, and most preferably below -20°C. Thus, it is preferred that the only treatment of the highly viscous liquid biological sample in the context of the present invention is freezing the sample.

A "nucleic acid isolation procedure" in the context of the present invention is a method that allows for isolation of nucleic acids from a complex mixture of substances and/or molecules such as a cell lysate. Preferably, the nucleic acid isolation procedure in the context of the present invention is based on a silica membrane or magnetic bead technology. Nucleic acid isolation kits are commercially available, for example, the EZ-1 DNA Tissue Kit (ordering number 953034) or the QIAamp DNA Blood Kit (ordering number 51104) from Qiagen. Depending on the nucleic acid isolation procedure or kit used it may be necessary to supplement the processed highly viscous liquid biological sample with further reagents before performing the nucleic acid isolation procedure. For example, for nucleic acid isolation using silica membrane purifications, e.g., using the QIAamp spin procedure from Qiagen, it is required to add a mixture of ethanol and Qiagen lysis buffer AL (80%/20%) to the sample before application of the sample to the nucleic acid isolation procedure. Most preferably, the nucleic acid isolation procedure contemplated by the present invention does not involve extraction with organic solvents such as phenol and/or chloroform or precipitation with alcohol such as ethanol or isopropanol. The isolated nucleic acid is preferably 90%, more preferably 95%, most preferably 99% free of any other macromolecular structures such as protein after performing the nucleic acid isolation procedure.

A "nucleic acid amplification method" in the context of the present invention is any molecular biological technique that is suitable for amplifying, i.e., multiplying, a nucleic acid, wherein the amplification may be linear or exponential. Examples for nucleic acid amplification methods are polymerase chain reaction (PCR), nucleic acid sequence-based amplification (NASBA), ligase chain reaction (LCR), strand displacement amplification (SDA), multiple displacement amplification (MDA), Q-beta replicase amplification, and loop-mediated isothermal amplification. The amplification method may be specific for a certain nucleic acid such as a specific gene or a fragment thereof, or may be universal such that all or a specific type of a nucleic acid, such as mRNA, is amplified universally. For example, the skilled person may design oligonucleotide primers which specifically hybridize to the nucleic acid of interest and use these primers in a PCR experiment.

A "nucleic acid analysis method" in the context of the present invention is any method that allows for detection and/or identification of a specific nucleic acid, wherein the term "detection" also comprises the quantitative determination of a nucleic acid. The detection and/or identification may be based on specific amplification, for example, by the amplification of a specific DNA fragment using oligonucleotide primers specific for said DNA fragment in the polymerase chain reaction. The skilled person is well aware of how to design oligonucleotide primers which specifically hybridize to the nucleic acid of interest. The detection and/or identification may also be achieved without amplification, for example, by sequencing the nucleic acid to be analyzed or by sequence specific hybridization, for example, in the context of a microarray experiment. Sequencing techniques and microarray based analysis are well known procedures in the field.

The nucleic acid to be isolated, amplified, or detected and/or identified may be DNA, such as genomic DNA or cDNA, or RNA, such as messenger RNA (mRNA) or ribosomal RNA (rRNA). Preferably, the nucleic acid is DNA. The skilled person is well aware of nucleic acid isolation, amplification, and analysis methods having regard to the general knowledge and the literature in the field.

The term "ready-to-use reaction tube" refers to pre-aliquoted reaction tubes that can be directly used for sample processing. This has the advantage that the reaction buffer does not have to be prepared and aliquoted before each use.

The materials and processes described herein are suited for a one-tube-processing of biological samples such as highly viscous biological samples.

The term "one-tube-processing" means that all processing steps are performed in one tube, omitting the need for further handling steps. The phrase "in one tube" means according to the invention that the processed sample or a part thereof containing the desired material such as a nucleic acid material is not transferred from one vessel to another vessel during the processing steps. However, the term "tube" according to the invention is meant to include all reaction vessels of a suitable size and shape. Preferably, "one-tube-processing" in the context of the present invention means "one-tube-liquefaction", more preferably "one-tube-lysis", i.e., all processing steps up to liquefaction and/or lysis, respectively, are performed in one vessel. Most preferably, the thus processed material can be directly applied to subsequent procedures such as nucleic acid isolation, amplification, analysis and/or detection procedures.

A "pathogen" in the context of the present invention relates to an infectious organism such as a virus, a bacterium, protozoa, yeast, fungi, or a parasite. Preferably, the pathogen in the context of the present invention is a bacterium, more preferably a human pathogenic bacterium. Preferably, the bacterium is of the family *Mycobacteriaceae,* i.e., a mycobacterium, most preferably *M. tuberculosis.*

In a first aspect, the present invention relates to a lysis buffer comprising, preferably consisting of, at least one chaotropic agent and at least one reducing agent, wherein the lysis buffer does not contain proteases, DNases, glycosidases, and lipases. Preferably, the lysis buffer according to the invention further comprises beads. Preferably said beads are made of a solid inert material such as glass, ceramics, or metal such as steel, most preferably the beads are made of glass. In a preferred embodiment, the nature and concentration of the chaotropic agent and/or the nature and concentration of the reducing agent is such that liquefaction of a highly viscous liquid biological sample is achieved when the lysis buffer is mixed with the sample, wherein preferably the highly viscous liquid biological sample is selected from the group consisting of sputum, pus, pleural fluid, gastric aspirate, endotracheal aspirate, transtracheal aspirate, bronchoalveolar lavage, laryngeal swab, and nasopharyngeal swabs, most preferably sputum. In a particular preferred embodiment, the highly viscous liquid biological sample is untreated sputum, i.e., raw sputum.

In a preferred embodiment of the lysis buffer according to the present invention, the chaotropic agent is guanidinium thiocyanate, guanidinium isothiocyanate, guanidinium hydrochloride, guanidinium chloride, alkali thiocyanate, alkali isothiocyanate, alkali iodide, or alkali perchlorate, wherein the alkali ion is preferably potassium or sodium. In a more preferred embodiment, the chaotropic agent is selected from the group consisting of guanidinium hydrochloride, guanidinium thiocyanate, and guanidinium isothiocyanate, wherein most preferably the chaotropic agent is guanidinium hydrochloride or guanidinium thiocyanate. The skilled person will recognize that more than one chaotropic agent may be combined in the lysis buffer according to the present invention. For example, guanidinium hydrochloride and guanidinium thiocyanate may be combined. In one embodiment, the chaotropic agent in the sense of the present invention is a commercially available lysis buffer which contains as a component a chaotropic agent, preferably as a main component. In this context, "main component" means that the buffer contains, besides water, primarily the chaotropic agent. An example for such a commercially available lysis buffer that is considered a chaotropic agent in the context of the lysis buffer according to the present invention is the Qiagen lysis buffer AL (Qiagen ordering number 19075) which contains guanidinium hydrochloride.

In a preferred embodiment, the chaotropic agent or the combination of chaotropic agents is present in a concentration in the range of 2 M to 8 M, e.g., in a concentration of 2 M, 2.5 M, 3 M, 3.5 M, 4 M, 4.5 M, 5 M, 5.5 M, 6 M, 6.5 M, 7 M, 7.5 M, or 8 M, preferably in a concentration of more than 4 M, even more preferably between 5 M and 6 M, and most preferably in a concentration of 5.5 M. However, the skilled person will recognize that the optimal concentration of the chaotropic agent in the lysis buffer according to the present invention will depend on the nature of the chaotropic agent and the sample to be processed and may be adjusted based on the chaotropic agent or the combination of chaotropic agents used. The most preferred chaotropic agents are guanidinium hydrochloride and guanidinium thiocyanate.

As described above, the chaotropic agent contained in the lysis buffer according to the present invention may be provided by a commercially available lysis buffer. In this context, the commercially available lysis buffer as described above is present in the lysis buffer according to the present invention preferably to at least 80% (vol/vol), more preferably to at least 85% (vol/vol), even more preferably to at least 90% (vol/vol), and most preferably to at least 95% (vol/vol), and is most preferably the Qiagen lysis buffer AL.

In a further preferred embodiment of the lysis buffer according to the present invention, the reducing agent is selected from the group consisting of dithiothreitol (DTT), N-acetyl-cysteine (NALC), beta-Mercaptoethanol, Tris(2-Carboxyethyl) phosphine (TCEP), and thioredoxin. Preferably, the reducing agent is NALC or DTT, most preferably DTT. A combination of more than one reducing agent is also contemplated. The reducing agent or combination of reducing agents may be present in the lysis buffer in a concentration in the range of 1 mM to 100 mM, e.g., in a concentration of 1 mM, 5 mM, 10 mM, 15 mM, 20 mM, 25 mM, 30 mM, 40 mM, 50 mM, 60 mM, 70 mM, 80 mM, 90 mM, or 100 mM, preferably the concentration is above 20 mM, more preferably about 50 mM. However, the skilled person will recognize that the concentration of the reducing agent in the lysis buffer according to the present invention will depend on the nature of the reducing agent and the sample to be processed and may be adjusted based on the reducing agent or combination of reducing agents used.

In a preferred embodiment, the beads, preferably the glass beads, in the lysis buffer according to the present invention have a diameter in the range of about 300 µm to 800 µm, e.g., a diameter of 300 µm, 400 µm, 500 µm, 600 µm, 700 µm, or 800 µm, and preferably have a diameter of about 600 µm. Preferably, the beads are acid washed to dissolve or hydrolyze any contaminants that may be present on the untreated beads. For example, beads, preferably glass beads, may be acid washed by soaking them for at least one hour in 2 M HNO₃ and rinsing them with water until the rinse water is no longer acidic. Alternatively, acid washed glass beads may be obtained commercially, for example, from Sigma-Aldrich (ordering number G8772). Preferably, the beads, preferably the glass beads, are present in an amount in the range of about 650 to 900 mg/ml, i.e., in an amount of about 650 mg/ml, 700 mg/ml, 750 mg/ml, 800 mg/ml, 850 mg/ml, or 900 mg/ml, most preferably in an amount of about 750 mg/ml. For example, 140 mg of glass beads may be added to 180 µl of lysis buffer to obtain an amount of 777 mg/ml glass beads in the lysis buffer (thus, the volume of the glass beads is not taken into account). The beads, preferably the glass beads, in this preferred embodiment of the lysis buffer according to the present invention are suitable for bead milling.

In a particular preferred embodiment, the lysis buffer is free of enzymes. In another preferred embodiment, the chaotropic agent in the lysis buffer is not urea. Furthermore, in a preferred embodiment, the reducing agent is not beta-Mercaptoethanol. In another preferred embodiment, the lysis buffer does not contain a detergent, in particular, the lysis buffer according to this embodiment does not contain N-lauroyl-sarcosine, Tween 20, Tween 80, and Triton-X-100. Furthermore, in another preferred embodiment, the lysis buffer according to the present invention does not contain a chelating agent, in particular, it does not contain EDTA. In a particularly preferred embodiment, the lysis buffer does not contain an enzyme and does not contain a detergent. In another particularly preferred embodiment, the lysis buffer does not contain an enzyme and does not contain a chelating agent. In a most preferred embodiment, the lysis buffer does not contain an enzyme, a detergent, and a chelating agent.

In a preferred embodiment, the lysis buffer has a pH that is approximately neutral. In a preferred embodiment, the lysis buffer has a pH in the range of 5.5 to 8, i.e., a pH of 5.5, 6, 6.5, 7, 7.5, or 8, preferably a pH of around 7.

In a preferred embodiment, the lysis buffer according to the present invention comprises a chaotropic agent in a concentration in the range of 4 to 6 M, preferably about 5.5 M, a reducing agent in a concentration in the range of 25 to 75 mM, preferably about 50 mM, and optionally beads, preferably glass beads, of a diameter in the range of 500 to 700 µM, preferably about 600 µM, in a concentration in the range of 700 to 800 mg/ml, preferably about 750 mg/ml. Preferably, the chaotropic agent is guanidinium hydrochloride or guanidinium thiocyanate and/or the reducing agent is DTT. Preferably, the beads, preferably glass beads, are present in the lysis buffer.

In a particularly preferred embodiment, the lysis buffer according to the present invention consists of a chaotropic agent in a concentration in the range of 4 to 6 M, preferably about 5.5 M, a reducing agent in a concentration in the range of 25 to 75 mM, preferably about 50 mM, and optionally beads, preferably glass beads, of a diameter in the range of 500 to 700 µM, preferably about 600 µM, in a concentration in the range of 700 to 800 mg/ml, preferably about 750 mg/ml. Preferably, the chaotropic agent is guanidinium hydrochloride or guanidinium thiocyanate and/or the reducing agent is DTT. Preferably, the beads, preferably glass beads, are present in the lysis buffer.

In a particularly preferred embodiment of the lysis buffer according to the present invention, the lysis buffer comprises 85 to 95% (vol/vol) Qiagen lysis buffer AL and 5% (vol/vol) 1 M DTT, and optionally glass beads, preferably the lysis buffer consists of about 95% (vol/vol) Qiagen lysis buffer AL and about 5% (vol/vol) 1 M DTT to which glass beads may or may not be added. For example, 170 µl ± 5 µl Qiagen lysis buffer AL, 10 µl ± 2 µl 1 M DTT, and optionally 140 mg ± 20 mg glass beads of about 600 µm diameter may be mixed for producing the lysis buffer according to the present invention, wherein preferably the glass beads are added.

A second aspect of the present invention relates to the use of the lysis buffer according to the first aspect of the present invention for processing a highly viscous liquid biological sample. The highly viscous liquid biological sample may be any highly viscous liquid biological sample, however, is preferably derived from an animal, preferably from a human. In a preferred embodiment of this aspect of the present invention, the highly viscous liquid biological sample is selected from the group consisting of sputum, pus, pleural fluid, gastric aspirate, endotracheal aspirate, transtracheal aspirate, bronchoalveolar lavage, laryngeal swab, and nasopharyngeal swabs. Preferably, the highly viscous liquid biological sample is sputum.

In a preferred embodiment, the highly viscous liquid biological sample is untreated before processing using the lysis buffer according to the first aspect of the invention. Most preferably, the highly viscous liquid biological sample is raw sputum which means untreated sputum. In another embodiment, the highly viscous liquid biological sample, preferably sputum, is frozen before processing using the lysis buffer according to the first aspect of the present invention, however, preferably the use according to the second aspect of the present invention does not comprise any other treatment step before processing.

In a preferred embodiment of the second aspect of the present invention, the processing comprises liquefaction of the highly viscous liquid biological sample, preferably liquefaction of sputum, most preferably liquefaction of raw sputum.

In one embodiment of the use according to the second aspect of the present invention, the lysis buffer is present in a ready-to-use reaction tube. In this embodiment, the highly viscous liquid biological sample is added to the ready-to-use reaction tube and processing is performed in said tube. Preferably, the entire processing happens in a single tube, such that further handling steps that may be accompanied by the risk of contamination are omitted.

In a particularly preferred embodiment of the use according to the second aspect of the present invention, after processing, the highly viscous liquid biological sample is suitable for being directly applied to a nucleic acid isolation procedure without the need for further processing such as extraction with organic solvents or precipitation before application to the nucleic acid isolation procedure. However, depending on the nucleic acid isolation procedure it may be necessary to supplement the processed highly viscous liquid biological sample with further reagents. Preferably, the nucleic acid purification procedure is based on silica membranes or magnetic bead technology. For example, commercially available nucleic acid isolation kits, such as the EZ-1 DNA Tissue Kit (magnetic bead technology) or the QIAamp DNA Blood Kit (silica membrane based) from Qiagen, may be used in the context of the present invention. For EZ-1 nucleic acid isolation the processed highly viscous liquid biological sample is directly placed into the BioRobot^{®} EZ-1 workstation and further processing is performed according to the manufacturer's instructions. For QIAamp nucleic acid isolation ethanol/Qiagen lysis buffer AL (80%/20%) is added to the processed highly viscous liquid biological sample according to the manufacturer's instructions and the mixture is transferred onto a QIAamp spin column. Further process steps are then performed as recommended by the manufacturer. Alternatively, for silica membrane based isolation a vacuum based setup (Curetis custom prototype) may be used. Preferably, an underpressure of at least 400 mbar, more preferably at least 500 mbar, and most preferably at least 600 mbar is applied for binding and washing steps in this experimental setup.

A third aspect of the present invention relates to a method for processing a highly viscous liquid biological sample, comprising, preferably consisting of, the steps of (a) mixing the sample with the lysis buffer according to the first aspect of the present invention, (b) optionally heating the mixture, and (c) optionally bead milling the mixture. The highly viscous liquid biological sample may be any highly viscous liquid biological sample, however, is preferably derived from an animal, and most preferably from a human. In a preferred embodiment of this aspect of the present invention, the highly viscous liquid biological sample is selected from the group consisting of sputum, pus, pleural fluid, gastric aspirate, endotracheal aspirate, transtracheal aspirate, bronchoalveolar lavage, laryngeal swab, and nasopharyngeal swabs. Preferably, the highly viscous liquid biological sample is sputum.

In a preferred embodiment, the highly viscous liquid biological sample is untreated before mixing the sample with the lysis buffer in step (a) of the method according to the third aspect of the present invention. Most preferably, the highly viscous liquid biological sample is raw sputum which means untreated sputum. In another embodiment, the highly viscous liquid biological sample, preferably sputum, is frozen before mixing it with the lysis buffer in step (a) of the method according to the third aspect of the present invention, however, preferably the method does not comprise any other treatment steps before step (a) other than freezing the sample. Thus, in a preferred embodiment, the only treatment of the highly viscous liquid biological sample, preferably the sputum sample, before mixing it with the lysis buffer is freezing the sample.

In a fourth aspect, the present invention provides a method for analyzing a highly viscous liquid biological sample, preferably sputum, comprising, preferably consisting of, the steps of (a) mixing the sample, preferably the sputum sample, with the lysis buffer according to the first aspect of the present invention, (b) optionally heating the mixture, (c) optionally bead milling the mixture, and (d) applying the mixture to a nucleic acid amplification/analysis method. Preferably, step (d) is preceded by a step wherein the mixture of step (a) and optionally (b) or (c) is subjected to nucleic acid isolation.

In a fifth aspect, the present invention provides a method for detecting the presence of a pathogen in a highly viscous liquid biological sample, preferably a sputum sample, comprising, preferably consisting of, the steps of (a) mixing the sample, preferably the sputum sample, with the lysis buffer according to the first aspect of the present invention, (b) optionally heating the mixture, (c) optionally bead milling the mixture, and (d) applying the mixture to a nucleic acid amplification/analysis method that is suitable for detection of said pathogen. Preferably, step (d) is preceded by a step wherein the mixture of step (a) and optionally (b) or (c) is subjected to nucleic acid isolation. A pathogen in the context of the present invention may be any organism that causes a disease in another organism. It may be a virus, a bacterium, yeast, fungi, protozoa, a parasite etc. Preferably, the pathogen in the context of the fifth aspect of the present invention is a bacterium, more preferably a bacterium of the family of *Mycobacteriaceae,* i.e., a mycobacterium. Furthermore, also mycobacteria that are not associated with a disease may be detected using the method according to the fifth aspect of the present invention. The following species of the family *Mycobacteriaceae* may be detected using the method according to the fifth aspect of the present invention: *M. abscessus*, *M. africanum*, *M. agr, M. aichiense*, *M. alvei*, *M. arosiense*, *M. arupense*, *M. asiaticum*, *M. aubagnense*, *M. aurum*, *M. austroafricanum*, *Mycobacterium avium complex (MAC)* (group of species which are a significant cause of death in AIDS patients; species in this complex include: *M. avium*, *M. avium paratuberculosis*, which has been implicated in Crohn's disease in humans and Johne's disease in sheep, *M. avium silvaticum*, *M. avium "hominissuis", M. colombiense), M. boenickei, M. bohemicum, M. bolletii, M. botniense, M. bovis, M. branderi*, *M. brisbanense, M. brumae, M. canariasense, M. caprae, M. celatum, M. chelonae, M. chimaera*, *M. chitae, M. chlorophenolicum, M. chubuense, M. conceptionense, M. confluentis*, *M. conspicuum, M. cookii, M. cosmeticum, M. diernhoferi, M. doricum, M. duvalii*, *M. elephantis, M. fallax*, *M. farcinogenes*, *M. flavescens*, *M. florentinum*, *M. fluoroanthenivorans*, *M. fortuitum, M. fortuitum subsp*. *acetamidolyticum*, *M. frederiksbergense*, *M. gadium*, *M. gastri*, *M. genavense*, *M. gilvum, M. goodii, M. gordonae*, *M. haemophilum, M. hassiacum, M. heckeshornense, M. heidelbergense, M. hiberniae*, *M. hodleri, M. holsaticum, M. houstonense, M. immunogenum, M. interjectum, M. intermedium, M. intracellulare, M. kansasii*, *M. komossense, M. kubicae*, *M. kumamotonense*, *M. lacus*, *M. lentiflavum*, *M. leprae* (which causes leprosy), *M. lepraemurium, M. madagascariense*, *M. mageritense, M. malmoense, M. marinum, M. massiliense, M. microti, M. monacense, M. montefiorense, M. moriokaense*, *M. mucogenicum, M. murale, M. nebraskense*, *M. neoaurum, M. neworleansense*, *M. nonchromogenicum, M. novocastrense*, *M. obuense, M. palustre*, *M. parafortuitum*, *M. parascrofulaceum*, *M. parmense*, *M. peregrinum, M. phlei, M. phocaicum*, *M. pinnipedii, M. porcinum, M. poriferae*, *M. pseudoshottsii, M. pulveris, M. psychrotolerans*, *M. pyrenivorans*, *M. rhodesiae*, *M. saskatchewanense*, *M. scrofulaceum*, *M. senegalense*, *M. seoulense, M. septicum, M. shimoidei, M. shottsii, M. simiae, M. smegmatis*, *M. sphagni, M. szulgai, M. terrae, M. thermoresistibile, M. tokaiense*, *M. triplex, M. triviale*, *Mycobacterium tuberculosis complex (MTBC)* (members are causative agents of human and animal tuberculosis; species in this complex include: *M. tuberculosis,* the major cause of human tuberculosis, *M. bovis, M. bovis BCG, M. africanum*, *M. canetti, M. caprae, M. pinnipedii), M. tusciae, M. ulcerans,* which causes the "Buruli", or "Bairnsdale, ulcer", *M. vaccae*, *M. vanbaalenii*, *M. wolinskyi*, and *M. xenopi.* Preferably, the pathogen to be detected in the fifth aspect of the present invention is a bacterium of the family of *Mycobacteriaceae* which is selected from the group consisting of *M*. *tuberculosis, M. bovis, M. africanum*, *M. canetti, M. caprae*, *M. smegmatis,* and *M. pinnipedii.* Most preferably, the pathogen to be detected is *M. tuberculosis.*

In a preferred embodiment of the fourth and fifth aspect of the present invention, the highly viscous liquid biological sample is sputum, preferably untreated sputum. However, the skilled person will recognize that the highly viscous liquid biological sample may be any highly viscous liquid biological sample depending on the purpose of the analysis in the method of the fourth aspect of the present invention or the pathogen to be detected in the method of the fifth aspect of the present invention. Thus, any highly viscous liquid biological sample that is suitable for the detection of a pathogen is contemplated by the fifth aspect of the present invention.

In a preferred embodiment of the third, fourth, and fifth aspect of the present invention, the sample is mixed with the lysis buffer in step (a) in a volume ratio of lysis buffer to sample in the range of 1:1 to 1:2, e.g., in a ratio of 1:1, 1:1.2, 1:1.4, 1:1,6, 1:1.8, or 1:2, preferably 1:1.2. For example, 180 µl of lysis buffer, optionally containing 140 mg ± 20 mg beads, preferably glass beads, preferably with a diameter of about 600 µm, is mixed with 220 µl ± 30 µl of a highly viscous liquid biological sample, preferably a sputum sample, more preferably an untreated sputum sample. The skilled person will recognize that the exact aliquot amounts of a highly viscous liquid biological sample such as a sputum sample can be difficult if the specimens are very mucous, however, the system is not sensitive to some variation in sample input amount, thus, the variation in the sample volume may be higher. The components of the lysis buffer may be added separately to the highly viscous liquid biological sample, the lysis buffer may be pre-mixed and then added to the highly viscous liquid biological sample, or the sputum sample may be added to the pre-mixed lysis buffer. Preferably, the sputum sample is mixed with the pre-mixed lysis buffer contained in a ready-to-use reaction tube.

In a preferred embodiment of the third, fourth, and fifth aspect of the present invention, the sample is mixed with the lysis buffer in step (a) such that a good contact between the lysis buffer and the sample is guaranteed. The skilled person is well aware of how to achieve a thorough mixture of a highly viscous sample with a lysis buffer, for example, the sample may be added to the lysis buffer or vice versa and is then mixed by vortexing or pipetting up and down. If pipetting up and down is used, it may be advisable to use pipette tips which have a wide opening, for example, pipette tips of which the very tip has been cut off. In a preferred embodiment, the mixture of step (a) is incubated for a time period in the range of 5 minutes to 1 hour, e.g., 5, 10, 15, 20, 25, 30, 40, 50, or 60 minutes, preferably at around room temperature, e.g., at 18, 20, 22, 24, or 26°C. The incubation time depends on whether steps (b) and/or (c) are performed. The skilled person will recognize that when omitting step (b) or (c) or both the incubation time may be longer, for example, 60 minutes.

In a preferred embodiment of the third, fourth, and fifth aspect of the present invention, the lysis buffer is present in a ready-to-use reaction tube, preferably in a ready-to-use reaction tube according to the seventh aspect of the present invention. In this embodiment, the highly viscous liquid biological sample may be directly added to the ready-to-use reaction tube without having to prepare and aliquot the lysis buffer or having to add each buffer component individually to the sample.

In a preferred embodiment of the third, fourth, and fifth aspect of the present invention, the mixture is heated in step (b), preferably to a sufficient temperature and/or for a sufficient time to cause liquefaction of the sample, more preferably to cause lysis of the sample, i.e., the cells present in the sample. In a preferred embodiment, the heating step comprises inactivation of the cells in the sample, in particular, inactivation of mycobacteria. The skilled person can easily determine a suitable temperature and duration of heating to cause liquefaction by simple experiments determining the viscosity of the sample before and after heating treatments under varying conditions, i.e., varying temperatures and time periods. For example, the samples may be heated to 80°C for 5 minutes, 85°C for 5 minutes, 90°C for 5 minutes, 95°C for 5 minutes, 98°C for 5 minutes, 80°C for 10 minutes, 85°C for 10 minutes, 90°C for 10 minutes, 95°C for 10 minutes, 98°C for 10 minutes, 80°C for 15 minutes, 85°C for 15 minutes, 90°C for 15 minutes, 95°C for 15 minutes, or 98°C for 15 minutes, and the viscosity may be determined before and after the heating step. The skilled person will recognize that the optimal heating temperature and heating duration depends on the nature of the sample used. In a particularly preferred embodiment of the third, fourth, and fifth aspect of the present invention, the heating step comprises lysis of at least part of the cells, preferably all cells present in the sample. Most preferably, after the heating step, no viable cells, most preferably no viable mycobacteria, in particular no viable *M. tuberculosis*, is present in the mixture. The skilled person is well aware of how to determine whether viable bacteria are present in the mixture, for example, by cultivation tests. Preferably, the mixture is heated to a temperature in the range of 80°C to 99°C, e.g., to 80°C, 85°C, 90°C, 92°C, 94°C, 95°C, 96°C, 97°C, 98°C, or 99°C, more preferably the mixture is heated to at least 90°C, most preferably to about 96°C in step (b) of the method according to the third, fourth, or fifth aspect of the present invention. Preferably, the mixture is kept at the temperature it was heated to for a time period in the range of 5 to 30 minutes, e.g., for 5, 10, 15, 20, 25, or 30 minutes, more preferably for at least 10 minutes, most preferably for at least 15 minutes. In a particularly preferred embodiment of the third, fourth, and fifth aspect of the present invention, the mixture is heated to 96°C and kept at this temperature for 15 minutes. The skilled person will recognize that any combination of temperatures and time periods is contemplated by the present invention as long as the above described effects are achieved. For example, the samples may be heated to 90°C for 5 minutes, 90°C for 10 minutes, 90°C for 15 minutes, 90°C for 20 minutes, 90°C for 25 minutes, 90°C for 30 minutes, 96°C for 5 minutes, 96°C for 10 minutes, 96°C for 15 minutes, 96°C for 20 minutes, 96°C for 25 minutes, or 96°C for 30 minutes etc.

In another preferred embodiment of the third, fourth, and fifth aspect of the present invention, the mixture is bead milled in step (c). Bead milling means agitation of a sample in the presence of small beads. Preferably, the beads are made from a solid inert material such as glass, ceramics, or metal such as steel, most preferably they are made from glass, with a diameter of usually 0.2 - 1.0 mm. Bead milling results in homogenization of the sample and break up of cells due to the high liquid shear gradients and collision with the beads. The rate and effectiveness of the homogenization and cell lysis can be modified by changing the rates of agitation and the size of the beads, as well as the dimensions of the equipment. Preferably, the conditions of the bead milling step (c) of the method according to the third, fourth, or fifth aspect of the present invention are such that one or more physical properties of the mixture is different before and after the bead milling step. Preferably, the mixture is more homogenous after bead milling, more preferably the mixture is less viscous after bead milling, for example, at least by a factor of 2, and most preferably the bead milling comprises lysis of cells present in the mixture, most preferably lysis of bacterial cells. The skilled person is well aware of bead milling procedures. For example, bead mills in various dimensions are commercially available. Preferably, the beads used for the bead milling procedure in step (c) of the method according to the third, fourth, or fifth aspect of the present invention have a diameter in the range of about 300 µm to 800 µm, e.g., a diameter of 300 µm, 400 µm, 500 µm, 600 µm, 700 µm, or 800 µm, and preferably have a diameter of about 600 µm. Preferably, the beads, preferably the glass beads, are acid washed to dissolve or hydrolyze any contaminants. For example, beads, preferably glass beads, may be acid washed by soaking them for at least one hour in 2 M HNO₃ and rinsing them with water until the rinse water is no longer acidic. Alternatively, acid washed glass beads may be obtained commercially, for example, from Sigma-Aldrich (ordering number G8772). Preferably, the beads, preferably the glass beads, are present in an amount in the range of about 650 to 900 mg/ml, i.e., in an amount of about 650 mg/ml, 700 mg/ml, 750 mg/ml, 800 mg/ml, 850 mg/ml, or 900 mg/ml, most preferably in an amount of about 750 mg/ml. For example, 140 mg of acid washed glass beads may be added to 180 µl of lysis buffer to obtain an amount of 777 mg/ml glass beads in the lysis buffer (thus, the volume of the glass beads is not taken into account). Preferably, the mixture containing the beads, preferably the glass beads, is agitated at a speed in the range of 2500 to 3500 rpm, e.g., 2500, 2750, 3000, 3100, 3200, 3300, 3400, or 3500 rpm, most preferably at a speed of 3100 ± 100 rpm, preferably for a time period of at least 5 minutes.

In a particular preferred embodiment of the third, fourth, and fifth aspect of the present invention, the mixture is heated in step (b) as described above and bead milled in step (c) as described above. For example, the mixture may be heated to 96°C for 15 minutes in step (b) and then bead milled at 3100 ± 100 µm for at least 5 minutes in step (c). Any other combination of the above described heating and bead milling conditions is also contemplated. However, it is emphasized that the method according to the third, fourth, or fifth aspect of the present invention may omit the heating step (b) or the bead milling step (c) or both steps (b) and (c). The skilled person will recognize that if step (b) and/or step (c), in particular if both steps are omitted, the mixture is preferably incubated with the lysis buffer for a sufficient time to cause changes in one or more physical properties of the sample, preferably decreasing viscosity, for example, at least by a factor of 2, and most preferably for a sufficient time to cause lysis and/or inactivation of pathogens present in the sample. As described above, the skilled person is well aware of how to determine such changes in physical properties.

In a further embodiment of the third, fourth, or fifth aspect of the present invention, the method further comprises the step of isolating a nucleic acid from the mixture after step (c), or after step (b) if step (c) is not performed, or after step (a), if steps (b) and (c) are not performed. Most preferably, the beads which are optionally present in the mixture are removed before the mixture is applied to a nucleic acid isolation or amplification procedure. For example, the beads may be removed by letting the beads settle by gravity and transferring the supernatant to a fresh reaction tube. Most preferably, the mixture obtained after step (c), or after step (b) if step (c) is not performed, or after step (a), if steps (b) and (c) are not performed is suitable for being directly applied to a nucleic acid isolation procedure without the need for further processing such as extraction with organic solvents or precipitation before application to the nucleic acid isolation procedure. Thus, in a particularly preferred embodiment, the mixture is directly applied to a nucleic acid isolation procedure without any further processing such as extraction using organic solvents or precipitation before application to the nucleic acid isolation procedure. However, depending on the nucleic acid isolation procedure used it may be necessary to supplement the mixture with further reagents. Preferably, the nucleic acid purification procedure is based on a silica membrane or magnetic bead technology. For example, commercially available nucleic acid isolation kits such as the EZ-1 DNA Tissue Kit (magnetic bead technology) or the QIAamp DNA Blood Kit (silica membrane based) from Qiagen may be used in the context of the present invention. For EZ-1 nucleic acid isolation the processed highly viscous liquid biological sample, preferably the processed sputum, i.e., the mixture resulting from step (a), or optionally from steps (a) and (b), or optionally from steps (a) and (c), or optionally from steps (a), (b), and (c) is directly placed into the BioRobot^{®} EZ-1 workstation and further processing is performed according to the manufacturer's instructions. For QIAamp nucleic acid isolation ethanol/Qiagen lysis buffer AL (80%/20%) is added to the processed highly viscous liquid biological sample, preferably the processed sputum sample, according to the manufacturer's instructions and the mixture is transferred onto a QIAamp spin column. Alternatively, any other composition of Qiagen lysis buffer AL and ethanol or ethanol only (96 to 100%) may be added to the lysate to achieve a final percentage of sample to Qiagen lysis buffer AL to ethanol of approximately 1 : 1 : 1 prior to loading the mixture onto the silica membrane. Further process steps are then performed as recommended by the manufacturer. Alternatively, for silica membrane based isolation a vacuum based setup (Curetis custom prototype) may be used. Preferably, an underpressure of at least 400 mbar, more preferably of at least 500 mbar, and most preferably of at least 600 mbar is applied for binding and washing steps.

In a preferred embodiment of the fourth and fifth aspect of the present invention, the nucleic acid amplification/analysis method is selected from the group consisting of polymerase chain reaction (PCR), nucleic acid sequence-based amplification (NASBA), ligase chain reaction (LCR), strand displacement amplification (SDA), Q-beta replicase amplification, loop mediated isothermal amplification, multiple displacement amplification (MDA), and microarray analysis. However, it is noted that any other nucleic acid amplification/analysis method may be applied in the context of the present invention. Based on the general knowledge and the literature in the field, the skilled person is well aware of nucleic acid amplification/analysis methods and how to perform such methods. In a particular preferred embodiment, the nucleic acid amplification/analysis method is PCR.

Most preferably, the nucleic acid amplification procedure in the context of the fifth aspect of the present invention is suitable to detect, even more preferably to identify, the pathogen, wherein the pathogen is preferably a bacterium of the family of *Mycobacteriaceae,* preferably *M. tuberculosis.* For example, the nucleic acid obtained by the method according to the fifth aspect according to the present invention may be amplified by PCR using primers that are specific for the pathogen to be detected, e.g., the mycobacterium. The skilled person is well aware of how to design such specific primers based on the sequence of the pathogen genome which is generally publicly available, for example, on the National Center for Biotechnology Information (NCBI) homepage (http://www.ncbi.nlm.nih.gov).

In a particular preferred embodiment of the third, fourth, and fifth aspect of the present invention, the method comprises the liquefaction of the highly viscous liquid biological sample or the sputum, preferably comprises the lysis of the cells present in the sample, most preferably lysis and inactivation of pathogens, in particular mycobacteria, present in the sample. In a preferred embodiment of the method according to the third, fourth, and fifth aspect of the present invention, the highly viscous liquid biological sample, preferably the sputum, is not treated with proteases. In another preferred embodiment of the method according to the third, fourth, and fifth aspect of the present invention, the highly viscous liquid biological sample, preferably the sputum is not treated, in particular not cleared or decontaminated, before step (a). This means that the highly viscous liquid biological sample, preferably the sputum sample, is not treated with any agents such as DTT or NALC or a combination of these reagents to clarify/liquefy the sample or with SDS, NaOH, NALC, or a combination of these reagents to decontaminate the sample. "Decontamination" means that any organisms other than mycobacteria, in particular, *M. tuberculosis,* such as bacteria, yeast or host flora, is inactivated before the mixture is cultured to detect the presence of mycobacteria in the sample.

In a particular preferred embodiment, the method according to the third aspect of the present invention consists of the steps (a) mixing the highly viscous liquid biological sample, preferably the raw sputum sample, with the lysis buffer according to the first aspect of the present invention, wherein the lysis buffer contains beads, (b) heating the mixture, preferably to 96°C for 15 minutes, and (c) bead milling the mixture at 3100 ± 100 rpm for at least 5 minutes.

In a particular preferred embodiment, the method according to the fourth aspect of the present invention consists of the steps (a) mixing the highly viscous liquid biological sample, preferably the raw sputum sample, with the lysis buffer according to the first aspect of the present invention, wherein the lysis buffer contains beads, (b) heating the mixture, preferably to 96°C for 15 minutes, (c) bead milling the mixture, preferably at 3100 ± 100 rpm for at least 5 minutes, (d) optionally removing the beads, (e) isolating the nucleic acids from the mixture, and applying the nucleic acids to a nucleic acid amplification/analysis method, preferably PCR.

In a particular preferred embodiment, the method according to the fifth aspect of the present invention consists of the steps (a) mixing the raw sputum sample, with the lysis buffer according to the first aspect of the present invention, wherein the lysis buffer contains beads, (b) heating the mixture, preferably to 96°C for 15 minutes, (c) bead milling the mixture, preferably at 3100 ± 100 rpm for at least 5 minutes, (d) optionally removing the beads, (e) isolating the nucleic acids from the mixture, and applying the nucleic acids to a nucleic acid amplification/analysis method, preferably PCR, wherein, in the PCR, primers are used that allow for the detection of a mycobacterium, preferably *M*. *tuberculosis* or a mycobacterium present in the *Mycobacterium tuberculosis* complex.

In a particularly preferred embodiment of the method according to the third, fourth, and fifth aspect of the present invention, steps (a), (b), and (c) are performed in a single tube without the need of transferring supernatants or other handling steps requiring the removal of the sample/mixture from the reaction tube. Thus, preferably, the method according to the third aspect of the present invention is a "one-tube-processing" method. Preferably, these steps (a), (b), and (c) of the method according to the third, fourth, or fifth aspect of the present invention are performed in a ready-to-use reaction tube according to the seventh aspect of the present invention.

A sixth aspect of the present invention relates to a lysate of a highly viscous liquid biological sample comprising a highly viscous liquid biological sample and the lysis buffer according to the first aspect of the present invention. Preferably, said lysate is produced by the method according to the third aspect of the present invention, wherein preferably, steps (b) and/or (c), most preferably steps (b) and (c) are performed. In a preferred embodiment of the lysate, the highly viscous liquid biological sample is selected from the group consisting of sputum, pus, pleural fluid, gastric aspirate, endotracheal aspirate, transtracheal aspirate, bronchoalveolar lavage, laryngeal swab, and nasopharyngeal swabs, more preferably the highly viscous liquid biological sample is sputum. In a particularly preferred embodiment of the lysate according to the sixth aspect of the present invention, the highly viscous liquid biological sample, preferably the sputum, was untreated before mixing it with the lysis buffer. In a preferred embodiment, the only treatment of the highly viscous liquid biological sample, preferably the sputum sample, before mixing it with the lysis buffer is freezing the highly viscous liquid biological sample.

In a preferred embodiment of the sixth aspect of the present invention, the lysate is prepared by mixing the lysis buffer and the highly viscous liquid biological sample in a volume ratio in the range of 1:1 to 1:2, e.g., in a volume ratio of 1:1, 1:1.2, 1:1.4, 1:1,6, 1:1.8, or 1:2, preferably 1:1.2., wherein, if the lysis buffer contains glass beads, the volume of the lysis buffer is determined without the volume of the glass beads. In a preferred embodiment, the lysate is without glass beads, for example, because the lysis buffer did not contain glass beads or the glass beads have been removed from the lysate. Preferably, for producing the lysate the mixture of highly viscous liquid biological sample is heated and/or bead milled, preferably heated and bead milled as described above for the third, fourth, and fifth aspects of the present invention.

In a seventh aspect, the present invention provides a ready-to-use reaction tube containing the lysis buffer according to the first aspect of the present invention. Preferably, the tube can be securely closed such as a screw cap tube. Preferably, the tube, preferably the screw cap tube, has a volume in the range of 1 to 2 ml, preferably 1.5 ml. A 1.5 ml tube is preferred, since it can be readily used with standard heating blocks. However, depending on the nature and amount of sample to be processed/analyzed the volume of the tube may be adjusted. Preferably, the tube contains a maximal amount of lysis buffer of 1/4 of the volume of the tube, more preferably of 1/8 of the volume of the tube, wherein the volume of the lysis buffer is determined without the volume of the glass beads. In a most preferred embodiment, the ready-to-use reaction tube according to the seventh aspect of the present invention contains about 180 µl of the lysis buffer, wherein, if the lysis buffer contains glass beads, the volume of the lysis buffer is determined without the volume of the glass beads, preferably in a tube having a volume of 1.5 ml.

An eighth aspect of the present invention relates to a kit comprising the lysis buffer according to the first aspect of the present invention and an instruction leaflet containing instructions for using said lysis buffer for processing, preferably liquefying a highly viscous liquid biological sample. For example, the kit may contain an instruction leaflet describing the steps of the method according to the third, fourth, or fifth aspect of the present invention. In a preferred embodiment, the highly viscous liquid biological sample is sputum, preferably untreated sputum. In a particularly preferred embodiment, the lysis buffer is aliquoted in ready-to-use reaction tubes, preferably the kit comprises the ready-to-use reaction tubes according to the seventh aspect of the present invention. In a preferred embodiment, the kit further comprises means for DNA isolation, preferably a silica based or magnetic bead based nucleic acid purification matrix. Furthermore, the kit may further comprise disposable pipettes suitable for mixing the highly viscous liquid biological sample with the lysis buffer and transferring the lysate to a new tube.

The present invention provides a valuable alternative to existing approaches for the processing of highly viscous liquid biological samples, such as sputum, especially if isolation of nucleic acid and subsequent analysis thereof is required. Some of the advantages of the present invention are a low infection risk if highly pathogenic organisms are to be detected, efficient solubilization/liquefaction of highly viscous samples, reduced handling, and compatibility to different DNA isolation methods.

### EXAMPLES

### Example 1: Preparation of lysis buffers and sample processing

In the following, the lysis buffers used for testing and the steps for processing samples are described.

### Lysis buffers

- Lysis buffer 1:: 170 µl ± 5 µl Qiagen lysis buffer AL (containing up to 50% guanidinium hydrochloride according to the manufacturer) 10 µl ± 2 µl 1 DTT or 10% NALC or water optionally 140 mg ± 20 mg glass beads (600 µm, acid washed)
- Lysis buffer 2:: 170 µl ± 5 µl 6 M guanidinium hydrochloride
10 µl ± 2 µl 1 M DTT or water
- Lysis buffer 3:: 170 µl ± 5 µl 6 M guanidinium thiocyanate
10 µl ± 2 µl 1 M DTT or water
- Lysis buffer 4:: 170 µl ± 5 µl 6 M urea
10 µl ± 2 µl water

The buffer was aliquoted in 1.5 ml screw cap tubes. The tubes were stored between 15°C and 25°C for different time periods (cf. Example 3).

### Sample input

DTT- or NALC-treated sputum pools (comparative example) or raw sputum samples have been used as samples in the experiments described herein. However, mycobacterial suspensions in buffer (e.g., PBS) or growth medium can also be used as samples. In the experiments described herein, the sample volume was 220 µl ± 30 µl. It is noted that taking aliquot amounts from sputum samples can be difficult if the specimens are very mucous, however, the system is not sensitive to some variations in sample input amount, therefore upper and lower limits may be higher as for less viscous fluids. After adding the sample to the lysis tube, the tube was shortly vortexed to ensure good contact between sample and lysis buffer. All work with sputa suspected of containing *M. tuberculosis* have been performed under a laminar flow within an S3 laboratory (or directly at patient site). Filter tips were used for pipetting steps. Since it was dealt with highly viscous samples, slantwise cut filter tips with widened opening for pipetting aliquots from sputum samples were used.

### Heat inactivation

A commercially available heating block was used for the heat inactivation step (Eppendorf thermomixer). The heating time was at least 15 minutes. The heating temperature was 96°C ± 1°C. After removing the tubes from the heating block, the tubes were shortly vortexed to resolve any remaining bits of unliquefied sputum. After heat inactivation, the samples were transferred to an S 1 or S2 laboratory.

### Bead milling

Bead milling was optionally performed in a custom-made prototype lysator from Curetis at a speed of 3100 ± 100 rpm for at least 5 minutes.

### Supernatant transfer

After letting the beads settle, the supernatant was transferred into a fresh 1.5 ml screw cap tube using filter tips. For subsequent EZ-1 nucleic acid isolation the transfer volume was 200 µl ± 5 µl; for silica membrane nucleic acid isolation the transfer volume was 350 µl ± 5 µl. A transfer of up to 10 µl glass beads was tolerated.

### Further processing to the prelysed samples

For EZ-1 nucleic acid isolation the supernatant was directly used without any further treatment. For silica membrane nucleic acid isolation (QIAamp spin procedure or vacuum setup) 250 µl ± 5 µl ethanol/Qiagen lysis buffer AL (80%/20%) was added to the supernatant and shortly mixed by vortexing, then the mixture was transferred onto the silica membrane. Further processing was performed according to the manufacturer's instructions.

### Example 2: Comparison of different chaotropic agents in the lysis buffer

220 µl of untreated sputum sample were added to 180 µl lysis buffer 1 to 4 without glass beads (cf. Example 1). After short vortexing, the mixtures were heated for 15 min at 96°C. The mixtures were then again vortexed and liquefaction results were scored according to the scheme shown in Figure 1.

This experiment shows that chaotropic salts are suitable to liquefy untreated sputum samples (Figure 1). Efficiency and success varies between different agents and individual samples. Guanidinium thiocyanate exhibited the best liquefaction properties. Adding the reducing agent usually allows to achieve higher liquefaction efficiency, in particular, if guanidinium hydrochloride is used as chaotropic agent.

### Example 3: Storage effects on liquefaction efficiency

The concept of the "one-tube lysis assay" relies on prefilled ready-to-use tubes and requires storage of the final buffer desirably at ambient temperature. Therefore, liquefaction efficiency of long-term stored prefilled tubes containing lysis buffer 1 (containing either DTT or NALC, with or without glass beads) was examined with sputum aliquots. No detrimental effect was detected after 100 days of storage. Obviously, there was some variation in efficiency which did not change over time, but was caused rather by a variability of individual aliquots of the same (inhomogeneous) sputum sample or by variability in vortexing before heating when a good homogenization of sample and buffer is crucial for performance. An additional bead milling step (indicated in Figure 2) further improved efficiency by resolving or homogenizing any unlysed bits or clumps to provide required liquefaction qualities for subsequent DNA isolation steps (Figure 2).

### Example 4: DNA isolation from pretreated and raw sputum samples

### Pretreated sputum samples

Two sputum pools, i.e., "liquid pool" and "viscous pool", combined from 5 individual sputolysin-pretreated samples were spiked with 50000 or 10000 pathogens per milliliter (*Mycobacterium smegmatis* and *Staphylococcus aureus*) derived from freshly prepared suspensions in PBS. *M. smegmatis* was used as a model for *M. tuberculosis* with similar cell wall characteristics in order to circumvent the necessity of S3 laboratory work. Concentrations of bacterial suspensions were verified by plating aliquots of the PBS suspensions on agar plates and counting of colonies (Figures 3 and 4).

### Untreated raw sputum samples

Alternatively, individual raw untreated sputum samples spiked with *M. smegmatis* (25 000/ml) have been used (Figure 5).

### DNA isolation and sample testing

220 µl of sputum pools or individual raw sputum samples were transferred to pre-aliquoted lysis tubes containing 180 µl lysis buffer 1 with DTT and glass beads. After 15 min heating at 96°C and 5 min bead milling using a custom made Curetis prototype lysator, 200 µl of lysed supernatant were transferred to a fresh tube and further processed using an EZ-1 BioRobot^{®}. In parallel, tests using the QIAamp DNA isolation method were performed by transferring 350 µl of lysed supernatant to a new tube, followed by addition of 250 µl ethanol/Qiagen lysis buffer AL [80 % (vol/vol)/20 % (vol/vol)], short mixing and transfer to a QIAamp silica membrane.

After completion of DNA isolation, 1 to 3 µl of 200 µl DNA eluate were used for PCR tests using Hotstart Taq Polymerase (Qiagen) in 30 µl together with *M. smegmatis* or *S. aureus* specific primers, and amplified for 35 - 38 cycles. The molarity of amplified products was determined using an Agilent Bioanalyzer 9700.

All used individual sputum samples or pools, i.e., raw or sputolysin treated, were well liquefied using the "one-tube" lysis procedure according to the present invention without interference with subsequent nucleic acid isolation and nucleic acid amplification procedures such as PCR. The achieved sensitivities were for spiked mycobacteria (*Mycobacterium smegmatis*) or for a gram-positive test pathogen (*Staphylococcus aureus*) in a clinically relevant range of 10000 pathogens/ml or even lower (Figure 3). This is in the range of the sensitivity normally observed for detection with standard diagnostic techniques like smear microscopy and PCR from enriched pathogen fractions. Isolated DNA shows generally the typical spectrum of pure DNA (Figure 4). Furthermore molarities of amplified PCR products from spiked raw sputa are in a comparable range to molarities from a sputolysin treated sputum pool (Figure 5).

### Example 5: Comparison to classical diagnostic procedures of tuberculosis

To compare efficiencies and sensitivities of the "one-tube" liquefaction/lysis approach of the present application with current diagnostic procedures, a study with frozen sputum samples from tuberculosis-positive patients was performed. Sputum samples were selected with either a positive smear-microscopy or a positive PCR performed on enriched pathogen fractions after decontamination. 220 µl of the sputum sample was added to 180 µl lysis buffer 1 containing DTT and glass beads. After 15 min heating at 96°C and 5 min bead milling using a custom made Curetis prototype lysator, 200 µl of lysed supernatant were transferred to a fresh tube and further processed using an EZ-1 BioRobot^{®} according to the manufacturer's instructions. 1 to 3 µl of 200 µl DNA eluate were used for PCR tests using Hotstart Taq Polymerase (Qiagen) in 30 µl together with *Mycobacterium tuberculosis* complex (MTBC) specific or mycobacterium genus specific PCR primers directed against the 23S rRNA gene.

The vast majority of samples was readily detected positive for *Mycobacterium tuberculosis* complex using the "one-tube" lysis procedure according to the present invention, showing that the diagnosis based on the "one-tube" lysis procedure according to the present invention is equivalent to classical tuberculosis diagnostic procedures (Figure 6). Some few samples could not be detected or were close to the detection limit which could be explained either by storage (freezing, thawing effects) and by the fact that specimen leftovers had been used for this study which might miss already sputum compartments with high pathogen loads.

## Claims

1. A lysis buffer comprising at least one chaotropic agent and at least one reducing agent, wherein the lysis buffer does not contain proteases, DNases, glycosidases, and lipases.

2. The lysis buffer according to claim 1, further comprising beads.

3. The lysis buffer according to claim 1 or 2, wherein the nature and concentration of the at least one chaotropic agent and/or the nature and concentration of the at least one reducing agent is such that liquefaction of a highly viscous liquid biological sample is achieved when the lysis buffer is mixed with the sample.

4. The lysis buffer according to any one of claims 1 to 3, wherein the chaotropic agent is selected from the group consisting of guanidinium thiocyanate, guanidinium isothiocyanate, guanidinium hydrochloride, guanidinium chloride, alkali thiocyanate, alkali isothiocyanate, alkali iodide, and alkali perchlorate.

5. The lysis buffer according to any one of claims 1 to 4, wherein the reducing agent is selected from the group consisting of dithiothreitol (DTT), N-acetyl-cysteine (NALC), beta-Mercaptoethanol, Tris(2-Carboxyethyl) phosphine (TCEP), and thioredoxin.

6. Use of the lysis buffer according to any one of claims 1 to 5 for processing a highly viscous liquid biological sample.

7. A method for processing a highly viscous liquid biological sample, comprising the steps of
a. mixing the sample with the lysis buffer according to any one of claims 1 to 5,
b. optionally heating the mixture, and
c. optionally bead milling the mixture.

8. A method for analyzing a highly viscous liquid biological sample comprising the steps of
a. mixing the sample with the lysis buffer according to any one of claims 1 to 5,
b. optionally heating the mixture,
c. optionally bead milling the mixture, and
d. applying the mixture to a nucleic acid amplification/analysis method.

9. A method for detecting the presence of a pathogen in a highly viscous liquid biological sample comprising the steps of
a. mixing the sample with the lysis buffer according to any one of claims 1 to 5,
b. optionally heating the mixture,
c. optionally bead milling the mixture,
d. applying the mixture to a nucleic acid amplification/analysis method that is suitable for detection of said pathogen.

10. The method according to any one of claims 7 to 9, wherein the sample is untreated before mixing it with the lysis buffer in step a.

11. The method according to any one of claims 7 to 10 further comprising the step of isolating a nucleic acid from the mixture of step c.

12. The method of claim 11, wherein the mixture of step c is directly subjected to said nucleic acid isolation without any further processing such as extraction using organic solvents or precipitation.

13. A lysate of a highly viscous liquid biological sample comprising a highly viscous liquid biological sample and the lysis buffer according to any one of claims 1 to 5.

14. A ready-to-use reaction tube containing the lysis buffer according to any one of claims 1 to 5.

15. A kit comprising the lysis buffer according to any one of claims 1 to 5 and an instruction leaflet containing instructions for using said lysis buffer for liquefaction of a highly viscous liquid biological sample.

16. The lysis buffer according to any one of claims 3 to 5, the use according to claim 6, the method according to any one of claims 7 to 12, the lysate according to claim 13, or the kit according to claim 15, wherein the highly viscous liquid biological sample is selected from the group consisting of sputum, pus, pleural fluid, gastric aspirate, endotracheal aspirate, transtracheal aspirate, bronchoalveolar lavage, laryngeal swab, and nasopharyngeal swabs.
